# EUROPÄISCHE PATENTSCHRIFT

(11) **EP 3 258 985 B1**
(45) Veröffentlichungstag und Bekanntmachung des Hinweises auf die Patenterteilung: **03.04.2019**
(21) Anmeldenummer: 16705896.5
(22) Anmeldetag: 16.02.2016
(51) Int. Cl.: A61M 1/34, A61M 1/36

(54) **VORRICHTUNG ZUR DURCHFÜHRUNG EINER APHERESEBEHANDLUNG**
DEVICE FOR CARRYING OUT AN APHERESIS TREATMENT
DISPOSITIF DE RÉALISATION D'UN TRAITEMENT PAR APHÉRÈSE

(30) Priorität: 18.02.2015 DE 102015002073
(43) Veröffentlichungstag der Anmeldung: 27.12.2017
(73) Patentinhaber: Fresenius Medical Care Deutschland GmbH, 61352 Bad Homburg (DE)
(72) Erfinder: LEINENBACH, Hans-Peter, 3500 Krems (AT); GEBAUER, Frank, 3512 Mautern (AT); MAGER, Gerhard, 61352 Bad Homburg (DE); KLEWINGHAUS, Jürgen, 31440 Oberursel (DE)
(74) Vertreter: Herrmann, Uwe
(86) Internationale Anmeldenummer: PCT/EP2016/000262
(87) Internationale Veröffentlichungsnummer: WO 2016/131539

(56) Entgegenhaltungen:
- EP-A1- 0 414 006
- EP-A2- 0 111 696
- EP-A2- 0 263 384
- DE-A1- 10 157 569
- DE-C1- 4 338 858

## Beschreibung

Die vorliegende Erfindung betrifft eine Vorrichtung zur Durchführung einer Apheresebehandlung, wobei die Vorrichtung einen extrakorporalen Kreislauf aufweist, in dem sich ein regenerierbarer Einfach-Adsorber zur Abtrennung von Substanzen aus Blut oder zur Abtrennung von Substanzen aus mittels eines Plasmaseparators gewonnenen Plasma befindet, wobei eine Leitung zur Führung des Blutes oder des Plasmas vorgesehen ist, die sich zu dem Adsorber erstreckt und über die der Adsorber mit Blut oder Plasma beaufschlagt wird.

Aus dem Stand der Technik bekannte Adsorber sind Medizinprodukte, die zur Abreicherung von Blutplasmabestandteilen dienen. Diese bestehen üblicherweise aus einer Matrix, die sich in einem durchströmbaren Gehäuse befindet. Ein Rückhaltesystem stellt sicher, dass die Matrix, die z.B. als partikuläre Matrix ausgeführt sein kann, in dem Gehäuse verbleibt. Die Adsorbermatrix verfügt üblicherweise über eine Oberfläche, die speziell modifiziert ist, um das oder die gewünschte Zielmoleküle aus dem Blutplasma zu binden und somit aus dem Plasma entfernen zu können.

In der Immunapherese werden zur Zeit regenerierbare Adsorber zur Wiederverwendung sowie auch Einmaladsorber eingesetzt, die nur einmal verwendet und dann verworfen werden. Die Einmaladsorber werden nicht regeneriert und sind somit hinsichtlich ihrer Bindungskapazität limitiert. Diese Limitation ist insbesondere bei zu entfernenden Zielmolekülen störend, die in hohen Konzentrationen im Blutplasma, das im Rahmen der Erfindung auch einfach als Plasma bezeichnet wird, vorkommen. Um dem abzuhelfen, sind regenerierbare Adsorber bekannt. Bei deren Einsatz wird die Prozessierung des Plasmas unterbrochen und der Adsorber wird regeneriert, bevor die Prozessierung des Plasmas fortgesetzt wird.

Um keine Unterbrechung der Behandlung hinnehmen zu müssen, wird das sogenannte Doppelsäulenverfahren eingesetzt, bei dem die beiden Adsorbersäulen im Wechsel alternierend beladen und regeneriert werden. Somit kann eine kontinuierliche Prozessierung des Plasmas erfolgen und über die Anzahl der Regenerationszyklen kann die gewünschte Abreicherung des Zielmoleküls erreicht werden.

Regenerierbare Adsorber erfordern üblicherweise eine Abtrennung des Blutplasmas aus dem Vollblut. Diese Abtrennung erfolgt parallel zur immunapheretischen Plasmaprozessierung in einem zweiten verbundenen extrakorporalen Kreislauf, wobei das prozessierte Plasma kontinuierlich wieder zusammen mit der blutzellhaltigen Fraktion des Blutes in den Patienten reinfundiert wird.

Herkömmliche regenerierbare Adsorber der Doppelsäulensysteme werden wegen ihrer hohen Kosten mehrfach bei demselben Patienten wiederverwendet, was eine zwischenzeitliche Konservierung erfordert. Die Konservierungslösung(en) enthalten Inhaltsstoffe, wie u.a. Quecksilber in Thioemersal, von denen eine potentielle Gefährdung des Patienten ausgeht, wenn die Freispülung des Adsorbers vor Gebrauch unzureichend oder gar nicht ausgeführt wird. Die Befüllung des Adsorbers mit der Konservierungslösung und das Freispülen vor der nächsten Anwendung stellen zusätzliche Arbeitsbelastungen für den Anwender dar. Abgesehen davon ist die Konservierungslösung eine Umweltbelastung.

Abgesehen von diesen Nachteilen ist auch die Regelung des Blut- und des Plasmaflusses sowie die Regelung des Flusses der unterschiedlichen Lösungen vergleichsweise aufwändig. Die komplexe Steuerung stellt hohe Anforderungen an die Beherrschung der Risiken, die mit dem komplexen Therapieverfahren verbunden sind.

Einsäulen-Therapieverfahren bieten demgegenüber mehrere Vorteile. Gegenüber Doppelsäulenverfahren ist das Behandlungssystem weniger komplex und somit leichter in Akut-Behandlungsmaschinen integrierbar. Ein zusätzlicher Monitor zur Prozessierung der Säulen ist nicht erforderlich und das Verfahren wird sicherer, weil keine parallele Verarbeitung von Plasma und toxischen Lösungen, wie z.B. der Elutionslösung stattfindet.

Ein Nachteil der Einsäulen-Therapieverfahren besteht darin, dass während der Regeneration der Säule bzw. des Adsorbers keine Behandlung, d.h. keine Adsorption von Schadstoffen an dem Adsorber stattfinden kann. Somit verlängert sich die Gesamtbehandlungszeit um die Dauer der Regenerationsphasen. Während dieser Phasen entsteht kein therapeutischer Nutzen. Die Unterbrechung der Therapie während der Regenerationsphasen stellt eine Reduktion der Verfügbarkeit der Vorrichtung dar, was ggf. zur Folge hat, dass therapierelevante Substanzen nicht mit der erforderlichen Rate aus dem Blut des behandelten Patienten entfernt werden können. Bei akuten Krankheitsverläufen bedeutet dies eine Behandlungsverzögerung und damit eine Verzögerung der Verbesserung des Patientenzustandes durch die TA-Therapie (TA = therapeutische Apherese). Bei mobilen Patienten entsteht ein erheblicher Komfort-Nachteil.

Vorrichtungen zur Durchführung einer Apheresebehandlung mit regenerierbaren Einfach-Adsorbern sind z.B. aus DE 43 38 858 (C1), DE 101 57 569 (A1) und EP 0 414 006 (A1) bekannt.

Der vorliegenden Erfindung liegt die Aufgabe zugrunde, eine Vorrichtung der eingangs genannten Art dahingehend weiterzubilden, dass Zeitverluste und Verfügbarkeitseinschränkungen bei Einsäulen- bzw. Einfach-Behandlungsverfahren minimiert werden und im Idealfall der Zeitbedarf von Regenerationen des Adsorbers aus therapeutischer Sicht aufgehoben werden.

Diese Aufgabe wird durch eine Vorrichtung mit den Merkmalen des Anspruchs 1 gelöst. Danach ist vorgesehen, dass die Apherese-Vorrichtung zumindest ein Reservoir zur Aufnahme von Blut oder Plasma aufweist, das stromaufwärts des Adsorbers in der Leitung angeordnet ist oder stromaufwärts des Adsorbers mit der Leitung in Verbindung steht, und dass wenigstens ein Controller oder Schaltmittel vorgesehen ist, der/das derart ausgebildet ist, dass in wenigstens einem Schaltzustand des Controllers bzw. der Schaltmittel das Reservoir mit Blut oder Plasma befüllt wird und die Beaufschlagung des Adsorbers mit Blut oder Plasma unterbunden wird, wenn die Regenerationsphase des Adsorbers eingeleitet wird.

Im Falle der Verwendung eines Controllers arbeitet die Vorrichtung automatisch, d.h. es wird veranlasst durch den Controller die Befüllung des Reservoirs vorgenommen und die Beaufschlagung des Adsorbers unterbunden, wenn dem Controller signalisiert wird, dass die Regenerationsphase des Adsorbers eingeleitet wird. Eine solche Signalisierung kann nach Ablauf einer bestimmten Behandlungsphase, d.h. nach Ablauf einer bestimmten Beladungsdauer des Adsorbers, oder in Abhängigkeit anderer Parameter, wie beispielsweise in Abhängigkeit der gemessenen Konzentration der zu adsorbierenden Substanzen stromabwärts des Adsorbers erfolgen.

Von der Erfindung ist jedoch auch der Fall umfasst, dass ein durch einen Nutzer zu betätigendes Schaltmittel vorhanden ist. Wird festgestellt, dass die Regeneration des Adsorbers eingeleitet wird, betätigt der Nutzer die Schaltmittel, was zur Folge hat, dass z.B. Pumpen, Ventile oder dergleichen so betrieben werden, dass die Befüllung des Reservoirs erfolgt und die Beaufschlagung des Adsorbers mit Blut oder Plasma für die Dauer der Regeneration unterbleibt.

Der Controller bzw. die Schaltmittel stehen mit Mitteln, insbesondere mit einer oder mehreren Pumpen und/oder Ventilen, zur Steuerung des Blut- bzw. Plasmaflusses derart in Verbindung, dass die gewünschte Füllung des Reservoirs mit Blut oder Plasma und die Absperrung des Adsorbers gegenüber dessen Beaufschlagung mit Blut oder Plasma erfolgt.

Erfindungsgemäß ist vorgesehen, dass bei der Regeneration des Adsorbers mit einer Regenerationsflüssigkeit der Blutfluss bzw. die Plasmaseparation nicht unterbrochen, sondern wenigstens zeitweise fortgeführt wird, wobei das Blut bzw. das Plasma in einem Reservoir gesammelt wird, bis die Regeneration abgeschlossen ist und die Behandlung, d.h. die Adsorption in dem Adsorber fortgeführt werden kann.

Die grundlegende Idee ist somit die Fortführung der Plasmaerzeugung bzw. der Blutförderung während der Regenerationsphase des Adsorbers, der als Einsäulenadsorber oder als sonstiger Einfach-Adsorber ausgeführt ist. Darunter ist ein Adsorber zu verstehen, der im Unterschied zu Doppelsäulensystemen als einzelner Adsorber eingesetzt wird. Die Fortführung der Plasmaerzeugung bzw. der Blutförderung kann sich über die gesamte Dauer der Regenerationsphase des Adsorbers erstrecken oder über einen Teil der Regenerationsdauer, beispielsweise bis das Reservoir vollständig gefüllt ist.

Das Blut bzw. das gewonnene Plasma wird in dem Reservoir zwischengespeichert. In der anschließenden Behandlungsphase wird zusätzlich zu dem weiterhin kontinuierlich geförderten Blut oder zu dem weiterhin kontinuierlich gewonnenen Plasma das in dem Reservoir gespeicherte Blut oder Plasma bei entsprechend höherer Flussrate über den Adsorber geleitet.

Im Falle der Behandlung von Blutplasma ist das Reservoir stromabwärts der Plasmaquelle, d.h. im Allgemeinen zwischen der Plasmapumpe und dem Adsorber angeordnet. Im Falle der Behandlung von Vollblut ist das Reservoir zwischen dem arteriellen Zugang des Patienten und dem Adsorber angeordnet.

Der geschwindigkeitsbestimmende Schritt der TA-Verfahren ist die Plasmaerzeugungsrate. Maßgeblich ist dabei der Patientenzugang, d.h. der verfügbare Blutfluss und der ohne Verfahrensstörung sicher abtrennbare Plasmaanteil. Typischerweise werden bei der Plasmaerzeugung durch Hämofiltration Plasmaflussraten von 15 - 25 ml/min erreicht.

Die vorliegende Erfindung umfasst jede beliebige Art der Plasmaabtrennung, wie z.B. den Einsatz eines Plasmafilters, einer Zentrifuge etc..

Um den vergleichsweise hohen Volumenstrom bei der Entleerung des Reservoirs verarbeiten zu können, muss der Adsorber in der Lage sein, auch bei deutlich höheren Flüssen bestimmungsgemäß zu arbeiten. Die Bindungsvorgänge am Adsorber müssen hinreichend schnell sein, d.h. die Bindung zwischen dem Adsorber und der Substanz im Blut bzw. im Plasma muss eine ausreichend hohe Bindungskonstante besitzen. Zudem dürfen sich die Strömungseigenschaften, wie das Flussprofil, der Durchflusswiderstand und damit der resultierende Druckverlust über die Länge des Adsorbers bei höherer Flussrate nicht nennenswert verschlechtern. Im Einzelfall muss der Plasmafluss über den Adsorber auf einen Maximalwert limitiert werden.

Vorzugsweise ist der Adsorber in einem Leitungsabschnitt des extrakorporalen Kreislaufs angeordnet, in dem Blutplasma vorliegt, d.h. der Adsorber wird mit Blutplasma beaufschlagt. Dementsprechend ist in einer bevorzugten Ausgestaltung der Erfindung vorgesehen, dass ein erster extrakorporaler Kreislauf vorgesehen ist, in dem sich der Plasmaseparator befindet, an dessen Sekundärseite sich ein zweiter extrakorporaler Kreislauf anschließt, wobei das Reservoir und der Adsorber in dem zweiten extrakorporalen Kreislauf angeordnet sind und wobei eine Rücklaufleitung vorgesehen ist, die den Adsorber mit dem ersten extrakorporalen Kreislauf verbindet. Der erste extrakorporale Kreislauf steht über einen arteriellen und einen venösen Zugang mit dem Patienten in Verbindung und enthält einen Plasmaseparator, z.B. in Form eines Plasmafilters. Auf der Sekundärseite, d.h. auf der Seite des gewonnenen Plasmas befindet sich ein zweiter extrakorporaler Kreislauf, in dem sich das Reservoir und stromabwärts davon der Adsorber befinden.

Die vorliegende Erfindung ist jedoch nicht darauf beschränkt und umfasst auch Systeme, die einen mit Vollblut beaufschlagten Adsorber aufweisen.

In einer bevorzugten Ausgestaltung der Erfindung befindet sich das Reservoir zwischen der Plasmapumpe bzw. zwischen der ersten Plasmapumpe (hiermit ist die erste Pumpe im Plasmazweig nach der Plasmaabtrennung vom Vollblut gemeint) und dem Adsorber.

Die Anbindung des Reservoirs an den Flussweg des Blutes bzw. des Plasmas kann unterschiedlich erfolgen: denkbar ist es, dass das Reservoir von Blut oder Plasma durchflossen wird und somit wenigstens eine Zu- und wenigstens eine Ableitung aufweist.

Alternativ kann das Reservoir als Seitenzweig oder in einem Seitenzweig der das Blut bzw. das Plasma führenden Leitung angeordnet sein.

Alternativ kann das Reservoir in einem Leitungsabschnitt parallel zu der das Blut bzw. Plasma führenden Leitung angeordnet sein.

Der Controller bzw. das Schaltmittel kann derart ausgebildet sein, dass er/es die Plasmaseparation oder die Förderung des Vollblutes aussetzt, wenn das Maximalvolumen in dem Reservoir erreicht ist und der Regenerationszyklus noch nicht abgeschlossen ist.

Auch ist es denkbar, dass der Controller oder das Schaltmittel derart ausgebildet ist, dass er/es zunächst die Förderung des gesamten separierten Plasmas in das Reservoir veranlasst und auf einen Bypass um den Adsorber umschaltet, wenn das Maximalvolumen in dem Reservoir erreicht ist und der Regenerationszyklus noch nicht abgeschlossen ist. Der Bypass ist derart ausgebildet, dass dieser den Adsorber umgeht und das erzeugte Plasma über die Rückführleitung wieder dem ersten extrakorporalen Kreislauf und somit dem Patienten zurückgibt.

Auch ist es denkbar, dass der Controller oder das Schaltmittel derart ausgebildet ist, dass ein Anteil des separierten Plasmas in das Reservoir und zeitgleich eine teilweise Plasmarückführung vorgenommen wird. Ist das Maximalvolumen in dem Reservoir erreicht und der Regenerationszyklus noch nicht abgeschlossen, erfolgt eine vollständige Rückführung des separierten Plasmas.

Es können Mittel, vorzugsweise in Form von Pumpen und/oder Ventilen, zur Entleerung des Reservoirs vorgesehen sein, wobei der Controller oder das Schaltmittel mit den Mitteln zur Entleerung des Reservoirs in der Verbindung steht und diese aktiviert, wenn die Regenerationsphase des Adsorbers abgeschlossen ist. Die Entleerung des Reservoirs kann somit automatisch, d.h. veranlasst durch einen Controller oder durch die Schaltmittel, d.h. veranlasst durch einen Nutzer initiiert werden.

Die Mittel zur Befüllung des Reservoirs können durch eine stromabwärts des Plasmaseparators angeordnete Pumpe, durch die Blutpumpe, durch eine Pumpe, die in einer zu dem Reservoir führenden Leitung angeordnet ist, oder durch einen pneumatischen Aktor gebildet werden.

Handelt es sich bei Flüssigkeit, mit der der Adsorber beaufschlagt wird um Plasma, d.h. um Blutplasma, dient als Mittel zum Befüllen des Reservoirs vorzugsweise die Plasmapumpe, die unmittelbar auf den Plasmaseparator folgt, d.h. unmittelbar stromabwärts des Plasmaseparators angeordnet ist. Diese ist unabhängig davon erforderlich, ob ein Reservoir vorhanden ist. Sie dient zur Erzeugung eines definierten Plasmaflusses aus dem Plasmaseparator.

Grundsätzlich kann die Befüllung des Reservoirs auch durch eine andere Pumpe, z.B. durch eine in einem Seitenzweig des Blut- oder Plasmaflussweges angeordnete Pumpe erfolgen.

Denkbar ist auch eine pneumatische Befüllung des Reservoirs. So kann bei der Verwendung eines starren Hohlkörpers eine pneumatische Aktoreinheit angeschlossen werden, durch die in dem Hohlkörper ein Unterdruck erzeugt wird. Diese kann an die Belüftung des Hohlkörpers angeschlossen werden. Auch bei flexiblen oder elastischen Hohlkörpern kann die Pumpfunktion pneumatisch erfolgen, wenn diese in eine geräteseitig angeordnete, starre und gegen Umgebungsluft abdichtbare Aufnahme eingelegt werden.

Auch kann die Unterdruckerzeugung in dem Hohlkörper durch äußere mechanische Kräfte erfolgen, wie z.B. durch eine Spritze oder durch einen beiderseits mechanisch gefassten Faltenbalg.

Ferner kann vorgesehen sein, dass die Mittel zur Entleerung des Reservoirs durch die Schwerkraft, durch eine stromabwärts des Reservoirs angeordnete Pumpe, durch eine Pumpe, die in einer von dem Reservoir abführenden Leitung angeordnet ist, durch einen pneumatischen Aktor oder durch das elastische Material des Reservoirs gebildet werden.

An dieser Stelle wird darauf hingewiesen, dass die Begriffe "stromaufwärts" und "stromabwärts" die Anordnung in Bezug auf die Strömungsrichtung des Blutes bzw. des Plasmas während der Behandlung bzw. während der Befüllung und Entleerung des Reservoirs bezeichnen.

Im einfachsten Fall erfolgt die Entleerung durch Schwerkraft, indem das Reservoir in ausreichender Höhe über dem Adsorber angeordnet ist. Durch die Schwerkraft muss der Flusswiderstand des Adsorbers und der venöse Rückgabedruck aufgebracht werden. Alternativ oder zusätzlich zu der Nutzung der Schwerkraft kann ein elastischer Hohlkörper verwendet werden, der das Plasma bzw. das Blut durch Dehnung seiner Wandungen unter einen Vordruck setzt. Bei diesen Anordnung besteht der Vorteil einer vergleichsweise einfachen Anordnung, allerdings der Nachteil, dass der Fluss nicht oder nur begrenzt aktiv gesteuert werden kann.

Grundsätzlich können als regelbare Alternative oder Ergänzung zu den beschriebenen pneumatisch betriebenen Anordnungen Mittel zur Volumen-, Pegel- oder Flussmessung herangezogen werden.

Eine gesteuerte Entleerung ist beispielsweise mittels einer Pumpe möglich. Wird das Reservoir im Flussweg des Blutes bzw. des Plasmas angeordnet, wird diese Pumpe zwischen dem Reservoir und dem Adsorber angeordnet. Auch eine in einem Seitenzweig des Plasma- oder Blutflusses angeordnete Pumpe kann zur Entleerung des Reservoirs verwendet werden.

Auch kann das Entleeren des Reservoirs durch eine oder mehrere Dosierpumpen für Regenerationslösungen erfolgen. Ferner kann die Abgabe des Plasmas oder des Blutes durch mechanisch angekoppelte Ausführungsformen des Reservoirs erzielt werden.

Im Rahmen der vorliegenden Erfindung wird als "Pumpe" jede beliebige herkömmliche Pumpe sowie jedes andere zur Förderung von Flüssigkeiten geeignete Mittel verstanden.

Vorzugsweise ist die zur Entleerung dienende Pumpe zwischen dem Reservoir und dem Adsorber angeordnet. Grundsätzlich ist auch eine Anordnung dieser Pumpe stromabwärts des Adsorbers möglich und von der Erfindung mit umfasst.

Weiterhin kann vorgesehen sein, dass in der Leitung eine Pumpe angeordnet ist und dass sich das Reservoir zwischen der Pumpe und dem Adsorber befindet und/oder dass das Reservoir in der Leitung, in einer von der Leitung abzweigenden Leitung oder in einer zur Leitung parallel verlaufenden Leitung angeordnet ist.

Denkbar ist es, dass es sich bei dem Reservoir um einen starren Hohlkörper oder um einen Hohlkörper mit elastischen und/oder flexiblen Wandungen handelt.

Im einfachsten Fall handelt es sich um einen starren, flexiblen oder elastischen Körper mit einem oder mehreren Schlauchanschlüssen.

Ein starrer Hohlkörper kann als beliebig geformtes Hohlteil realisiert sein. Er verfügt vorzugsweise über eine Be- und Entlüftung, die z.B. mit einer Sterilmembran versehen ist. Der Abfluss befindet sich vorzugsweise an der tiefsten Stelle.

Handelt es sich um einen flexiblen Hohlkörper, kann dieser am einfachsten durch zwei miteinander verschweißte Folien in Form eines Beutels realisiert werden. Auch andere Konstruktionen, wie z.B. ein Faltenbalg sind realisierbar und von der Erfindung mit umfasst.

Ein elastischer Hohlkörper kann durch einen Beutel bestehend aus einer oder mehreren elastischen Folien realisiert werden, der das abgetrennte Plasma bzw. das Blut umschließt und sich mit dem aufgenommenen Volumen ausdehnt. Die Abgabe des Volumens erfolgt ausschließlich oder unterstützend aufgrund der Elastizität des Beutels durch das Zusammenziehen bei der Öffnung der Abgangsleitung.

Auch sind beliebige weitere technische Lösungen für Reservoire aus Kombinationen der vorgenannten Konstruktionsprinzipien mit ganz oder teilweise flexiblen und/oder elastischen Behälterwänden denkbar.

Das Reservoir kann über eine Be- und Entlüftung verfügen. Denkbar ist es weiterhin, dass das Reservoir über einen Abfluss und über einen Zufluss verfügt, die durch getrennte Elemente oder durch ein und dasselbe Element gebildet werden.

Das Reservoir kann ein Volumen zwischen 10 ml und 1000 ml aufweisen.

Das Reservoir kann das aufgenommene Volumen speichern und dieses auch wieder vollständig abgeben.

Vorzugsweise weist die Vorrichtung Mittel zur Steuerung oder Regelung des Plasma- oder Blutflusses auf. Diese sind beim Befüllen und beim Entleeren des Reservoirs erforderlich, da diese einen direkten Einfluss auf die Flüssigkeitsbilanz des Patienten haben und außerdem direkt den Fluss über den Adsorber beeinflussen. Die Mittel sind auch während der Behandlungsphase, in der das Blut oder Plasma über den Adsorber geleitet wird, erforderlich, um eine bestimmte Flussrate einstellen zu können.

Durch eine sinnvolle Steuerung oder Regelung des zusätzlichen Plasma- oder Blutflusses aus dem Reservoir kann eine Optimierung des prozessierten Gesamtflusses und der Behandlungszeit stattfinden. Vorzugsweise sind Kontrollmittel vorhanden, die ein Leerlaufen und ein Überfüllen des Reservoirs mit den damit verbundenen unerwünschten Effekten verhindern. Denn bedingt durch Fertigungstoleranzen von Pumpschläuchen kann eine Abweichung der Ist- von der Sollförderrate vorliegen oder es können mit dem Auftreten von Druckunterschieden Abweichungen aufgrund der Fördercharakteristik der Pumpen auftreten.

Vorzugsweise weist die Vorrichtung Mittel zur Erfassung des aktuell in dem Reservoir gespeicherten Volumens sowie der Zu- und Abflussrate in das bzw. aus dem Reservoir auf.

Vorzugsweise ist ein Drucksensor zwischen dem Plasmaseparator und der ersten Plasmapumpe und/oder ein Drucksensor am Adsorbereingang vorgesehen. Alternativ oder zusätzlich kann ein Drucksensor am Reservoir oder vor oder nach dem Reservoir angeordnet sein.

Weiterhin sind vorzugsweise Mittel zur Bestimmung bzw. Beschränkung der Menge des in dem Reservoir befindlichen Blutes oder Plasmas vorgesehen. Diese dienen insbesondere zur Limitierung des im dem Reservoir gespeicherten Volumens. Diese Kontrolle kann durch den Anwender erfolgen.

Vorzugsweise sind jedoch Mittel vorgesehen, die diese Aufgabe übernehmen und in Zusammenwirken mit dem Controller sicherstellen, dass die Menge einen Maximalwert nicht übersteigt.

Vorzugsweise ist der Controller derart ausgebildet, dass ein vollständig automatisierter Betrieb der Vorrichtung erfolgt. Der Controller ist vorzugsweise mit den Mitteln zur Steuerung des Fluidflusses verbunden und betreibt diese vorzugsweise selbsttätig, so dass ein automatisches Befüllen und Entleeren des Reservoirs, ein automatisches Beaufschlagen des Adsorbers mit Blut oder Plasma während der Behandlungsphase sowie eine automatische Regeneration des Adsorbers erfolgen.

Besteht das Reservoir aus einem flexiblen, aber nicht elastischen Material nimmt bei Überschreiten des Füllvolumens der Fülldruck wesentlich zu. Dies kann durch eine Druckbegrenzung in der Füllpumpe oder durch einen gesonderten Sensor überwacht werden.

Im Falle eines elastischen oder flexiblen Materials des Reservoirs könnte das Reservoir von einem festen Gehäuse zur Volumenbegrenzung umgeben sein. In diesem Fall und im Falle der Verwendung eines starren Reservoirs ergibt sich eine konstruktive Begrenzung des Maximalvolumens, das in dem Reservoir aufgenommen werden kann.

Denkbar ist es, dass die Mittel durch einen Sensor, insbesondere durch einen Drucksensor, durch einen optischen Sensor, durch einen Ultraschallsensor, durch einen Wegaufnehmer, durch ein festes Gehäuse zur Volumenbegrenzung, durch eine Waage oder durch eine Flussmesseinrichtung zur Messung des Zuflusses und des Abflusses aus dem Reservoir gebildet werden.

Auch beliebige andere Sensoren bzw. Messwertaufnehmer, die zur Erfassung des Volumens, der Masse oder des Füllstandes geeignet sind, kommen in Betracht und sind von der Erfindung mit umfasst.

Auch die Bestimmung des in dem Reservoir aufgenommenen Volumens durch die Messung von Zu- und Abflüssen ist von der Erfindung umfasst. Dazu können Flusssensoren bzw. Flussregler eingesetzt werden.

Auch eine mechanische Füllstandsüberwachung, z.B. ein Wegaufnehmer für die Beutelausdehnung ist denkbar.

Das Füllvolumen kann auch durch technisch übliche Mittel, wie z.B. durch optische Sensoren, Ultraschallsensoren etc. überwacht bzw. gemessen werden.

Auch kann das Reservoir unabhängig von der Ausführungsform und der Anordnung im Plasma- oder Blutflussweg an einer Waage positioniert werden. Dies hat den Vorteil, dass an das Material und an die Gestaltung des Reservoirs nur geringe Anforderungen gestellt werden müssen und dass durch die Wägung eine präzise und direkte Überwachung des Plasma- oder Blutvolumens im Reservoir möglich ist. Gleichzeitig können die Zu- und Abflüsse an Plasma und Blut jederzeit erfasst werden und können zur Verfahrenssteuerung oder - regelung verwendet werden.

In einer weiteren Ausgestaltung der Erfindung ist eine Bypassleitung um den Adsorber vorgesehen ist. Durch diese Leitung kann der Plasma- oder Blutfluss um den Adsorber geleitet werden, wenn das Reservoir vollständig gefüllt ist und die Plasmaseparation bzw. die Förderung des Vollblutes nicht unterbrochen werden soll. Dementsprechend kann der Controller oder die Schaltmittel derart ausgebildet sein, dass dieser/diese die Förderung des Blutes oder des Plasmas durch die Bypassleitung veranlasst, wenn die maximale Füllmenge in dem Reservoir erreicht ist, die Regeneration des Adsorbers aber noch nicht abgeschlossen ist.

Der Controller bzw. die Schaltmittel können auch derart ausgebildet sein, dass dieser/diese die Aussetzung des Betriebes der Blutpumpe oder des Plasmaseparators veranlasst, wenn die maximale Füllmenge in dem Reservoir erreicht ist.

In einer bevorzugten Ausgestaltung der Erfindung erfordert die technische Umsetzung der erfindungsgemäßen Idee ein Reservoir für Blut oder Plasma mit ausreichender Größe im Schlauchsystem, Vorrichtungen zum Füllen und zum Entleeren des Reservoirs, Maßnahmen zur Steuerung und Überwachung des zusätzlichen Plasmaflusses beim Füllen und beim Entleeren des Reservoirs, Vorrichtungen zur Überwachung des Füllstandes des Reservoirs und Maßnahmen zur sicheren Handhabung der Vorrichtung durch den Anwender.

Die vorliegende Erfindung betrifft des Weiteren ein Verfahren zum Betreiben einer Anordnung mit wenigstens einem Einfach-Adsorber zur Abtrennung von Substanzen aus Blut oder aus in einem Plasmaseparator gewonnenen Plasma, wobei während der Regenerationsphase des Adsorbers Blut oder Plasma nicht dem Adsorber zugeführt wird, sondern in einem Reservoir zwischengespeichert wird und wobei das zwischengespeicherte Blut oder Plasma nach der Regenerationsphase des Adsorbers dem Adsorber zugeführt wird.

Denkbar ist es, dass die Bereitstellung des Blutes oder des Plasmas unterbrochen wird, wenn das Reservoir einen maximalen Füllstand erreicht hat.

In einer möglichen Ausgestaltung der Erfindung ist vorgesehen, dass das Blut oder das Plasma in einem Bypass um den Adsorber geführt wird, wenn das Reservoir einen maximalen Füllstand erreicht hat. In diesem Fall ist keine Unterbrechung der Bereitstellung des Blutes oder des Plasmas vorgesehen.

Weitere bevorzugte Merkmale des Verfahrens sind Merkmale der oben beschriebenen Vorrichtung zur Durchführung eines Aphereseverfahrens. In einer möglichen Ausgestaltung bezieht sich das erfindungsgemäße Verfahren auf ein Verfahren zum Betreiben einer erfindungsgemäßen Vorrichtung.

Weitere Einzelheiten und Vorteile der Erfindung werden anhand eines in der Zeichnung dargestellten Ausführungsbeispiels näher erläutert. Es zeigen:

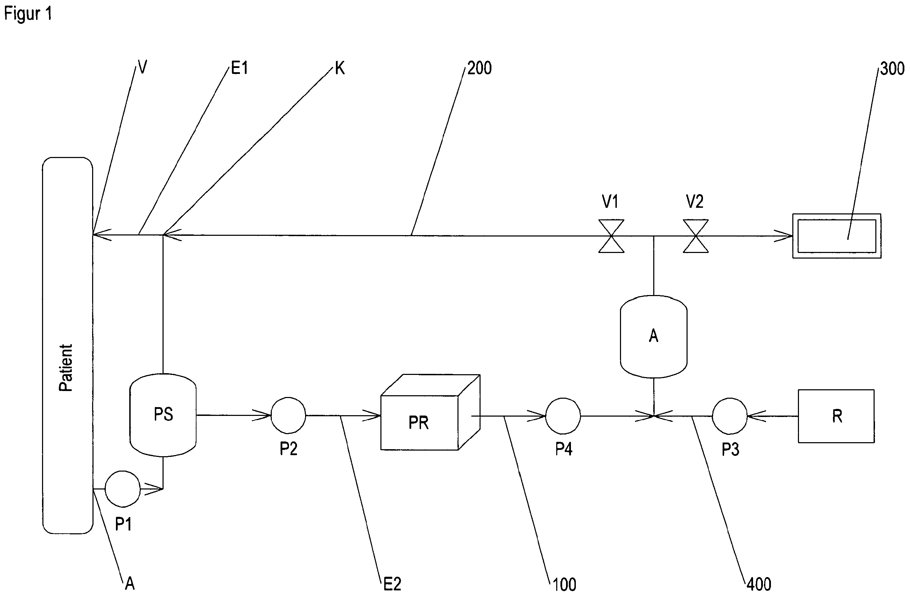

Figur 1 - 8: unterschiedliche Flussschemata einer erfindungsgemäßen Vorrichtung.
Gleiche oder funktionsgleiche Elemente der Apherese-Vorrichtung gemäß der Erfindung werden in den Figuren durch gleiche Bezugszeichen gekennzeichnet.

Figur 1 zeigt ein erstes Flussschema der erfindungsgemäßen Anordnung.

Mittels der Blutpumpe P1, die in dem ersten extrakorporalen Kreislauf E1 angeordnet ist, wird Blut aus einem arteriellen Zugang A des Patienten entfernt und dem Plasmaseparator PS zugeführt: Dabei kann es sich beispielsweise um einen Plasmafilter, eine Zentrifuge etc. handeln.

Der im Rahmen der Erfindung verwendete Ausdruck "Plasmafilter" oder "Plasmaseparator" umfasst jede beliebige Einrichtung, mittels derer Blutplasma aus dem Blut des Patienten gewonnen werden kann.

Die in dem Plasmaseparator PS nicht abgetrennten Blutbestandteile werden durch den ersten extrakorporalen Kreislauf E1 dem Patienten mittels eines venösen Zugangs V wieder zugeführt.

Das in dem Plasmaseparator PS abgetrennte Plasma wird mittels der Plasmapumpe P2 durch den zweiten extrakorporalen Kreislauf E2 in das Reservoir PR gefördert. Das Reservoir PR befindet sich in der ersten Leitung 100. Aus dem Reservoir PR fördert die Pumpe P4 das Plasma in den Adsorber A. Wie aus Figur 1 ersichtlich, befindet sich die Pumpe P2 stromaufwärts des Reservoirs PR und die Pumpe P4 stromabwärts des Reservoirs PR. Das in dem Adsorber A gereinigte Plasma gelangt bei geöffnetem Ventil V1 durch die Rückführleitung 200 zurück zum extrakorporalen Kreislauf E1und wird dort mit dem im Plasmaseparator nicht abgetrennten Teil des Blutes gemischt. Diese Mischung wird über den venösen Zugang V dem Patienten zugeführt.

Das Bezugszeichen R kennzeichnet ein Reservoir für eine Regenerationslösung, die zur Regeneration des Adsorbers A für eine bestimmte Zeitdauer über den Adsorber A geführt wird. Die verbrauchte Regenerationslösung wird bei geöffnetem Ventil V2 und geschlossenem Ventil V1 einem Abfallbehälter 300 oder einer sonstigen Abfallaufnahme zugeführt.

Das Bezugszeichen K kennzeichnet die Verbindungsstelle der beiden extrakorporalen Kreisläufe E1 und E2.

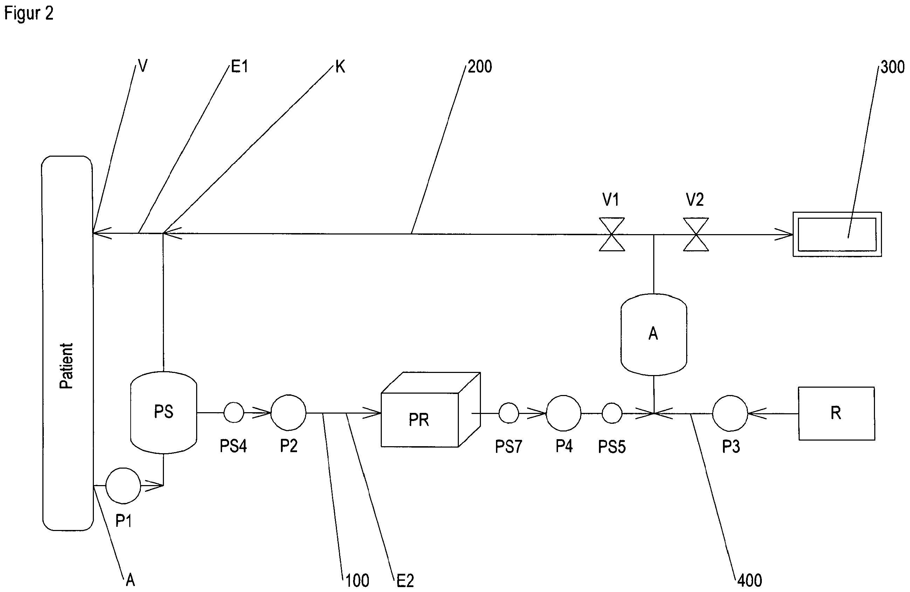

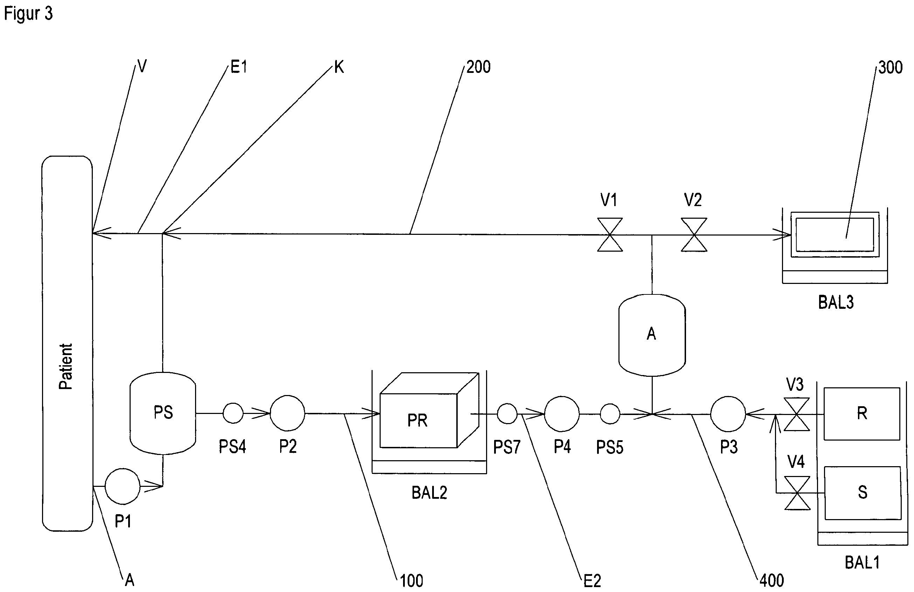

Die Anordnung gemäß Figur 2 unterscheidet sich von der Anordnung gemäß Figur 1 dadurch, dass sich zwischen dem Plasmaseparator PS und der Plasmapumpe P2, zwischen dem Reservoir PR und der Plasmapumpe P4 und zwischen letzterer und dem Adsorber A jeweils Drucksensoren PS4, PS7 und PS5 befinden, auf deren Funktion im Rahmen der Erläuterungen zu Figur 3 näher eingegangen wird.

Wie dies aus Figur 3 hervorgeht, wird Blut von dem Patienten mit der Pumpe P1 aus einem arteriellen Zugang A und einer arteriellen Leitung des ersten extrakorporalen Kreislaufs E1 entzogen und über den Plasmaseparator z.B. in Form eine Plasmafilters PS geführt. Von der Sekundärseite des Plasmafilters PS, auf der das gewonnene Plasma vorliegt, erstreckt sich der zweite extrakorporale Kreislauf E2, der an der Stelle K mit dem ersten extrakorporalen Kreislauf E1 verbunden ist.

Die auf der Sekundärseite des Plasmafilters PF in der Leitung 100 (im Folgenden auch als "Plasmaleitung" bezeichnet) angeordnete Pumpe P2 erzeugt einen Unterdruck, durch den das Plasma im Plasmafilter PF zu einem vorgegebenen Anteil vom Blut des Patienten abgetrennt wird. Der Drucksensor PS4 dient zur Messung des durch die Pumpe P2 erzeugten Unterdrucks.

Das im Plasmafilter PF abgetrennte Plasma wird ebenfalls durch die Pumpe P2 in das Reservoir PR gefördert, das sich ebenfalls in der Plasmaleitung 100 befindet. In dem Ausführungsbeispiel ist das Reservoir als ein Folienbeutel mit einem Zufluss und mit einem Abfluss ausgebildet. Grundsätzlich kommen auch beliebige anders ausgeführte Reservoirs in Frage und sind von der Erfindung umfasst.

Das Fassungsvermögen des Reservoirs liegt beispielsweise bei 200 bis 1000 ml, vorzugsweise bei 500 ml. Dem Folienbeutel kommt außer der Aufnahme des Plasmas keine weitere Funktion zu.

Das Reservoir PR ist auf einer Waage BAL2 angeordnet. Dadurch ist es möglich, kontinuierlich die Masse bzw. den Füllstand oder das Volumen des in dem Reservoir PR befindlichen Fluids zu ermitteln und zu überwachen. Die im dem Reservoir PR Menge des Plasmas bzw. dessen Volumen und Masse sind somit jederzeit bekannt. Dies ermöglicht es, durch geeignete Verfahrenssteuerung das maximal dem Patienten intermittierend, d.h. während der Regenerationsphasen des Adsorbers A, entzogene Plasmavolumen zu limitieren. Dazu kann ein Maximalwert in der Software der Vorrichtung hinterlegt sein, der Maximalwert kann aus den Patientendaten von der Vorrichtung berechnet werden oder es kann sich bei dem Maximalwert um eine Kombination der beiden vorgenannten Optionen handeln.

Die Kenntnis der Menge des in dem Reservoir PR befindlichen Plasmas bzw. des Füllstandes des Reservoirs ermöglicht eine automatische Steuerung und Optimierung des Plasmaflusses sowohl hinsichtlich des Eingangsflusses in das Reservoir als auch hinsichtlich des Ausgangsflusses aus dem Reservoir.

Der Drucksensor PS7, der in der Plasmaleitung stromabwärts des Reservoirs PR angeordnet ist, dient dazu, eine grobe Fehlfunktion, wie z.B. die Okklusion des Beutelabflusses oder die Überfüllung des Beutels über das Nennvolumen hinaus, festzustellen. Wird die Waage BAL2 sicher ausgelegt, kann auf den zusätzlichen Drucksensor PS7 auch verzichtet werden.

Die stromabwärts des Reservoirs PR in der Plasmaleitung befindliche Pumpe P4 fördert das Plasma aus dem Reservoir PR heraus und leitet dieses sodann über den Adsorber A. Der Drucksensor PS5 ist unmittelbar stromaufwärts des Adsorbers angeordnet und überwacht den Eingangsdruck des Adsorbers. Über das stromabwärts des Adsorbers befindliche geöffnete Ventil V1 wird das gereinigte Plasma durch die Rückführleitung 200 wieder zu dem extrakorporalen Kreislauf E1 geleitet und mit diesem zusammen dem Patienten zugeführt.

In die Plasmaleitung 100 mündet stromaufwärts des Adsorbers A die Leitung 400, die einerseits mit einem Reservoir R für Regenerationslösung zur Regeneration des Adsorbers A und andererseits mit einem Reservoir S für eine Spüllösung in Verbindung steht. Beide Reservoirs R und S sind jeweils durch Ventile V3 und V4 absperrbar. Die Förderung der Lösungen aus den Reservoirs R und S erfolgt mittels der Pumpe P3.

Das Bezugszeichen 300 kennzeichnet die Aufnahme für den Abfall, d.h. für zu verwerfende Lösungen.

Der Betrieb der in Figur 3 dargestellten Anordnung gestaltet sich wie folgt:
Je nach Beladungszustand des Adsorbers wird unterschieden zwischen einer Behandlungsphase, in der der Adsorber mit Substanzen aus dem Plasma beladen wird, und einer Regenerationsphase, in der der beladene Adsorber regeneriert wird. Während der Behandlungsphase wird der Adsorber aus dem Plasma beladen. Die Pumpe P3 steht und das Ventil V2 ist geschlossen.

Zu Beginn der Behandlung, d.h. in einer ersten Behandlungsphase, fördern die Pumpen P2 und P4, die sich stromaufwärts und stromabwärts des Reservoirs PR in der Leitung 100 befinden, mit gleicher Flussrate, was zur Folge hat, dass in dem Reservoir kein Plasmavolumen angesammelt wird.

Wird festgestellt, dass die Aufnahmekapazität des Adsorbers A erschöpft ist, wird die Regenerationsphase des Adsorbers A eingeleitet.

Dazu wird die Pumpe P4 gestoppt und die Pumpe P2 läuft weiter, u.U. mit gegenüber der Behandlungsphase reduzierter Flussrate. Da die Pumpe P4 steht, wird das erzeugte Plasma vollständig in das Reservoir PR gefördert. Die Überwachung der erzeugten Menge erfolgt beispielsweise gravimetrisch, d.h. mittels der Waage BAL2. Wird der Maximalwert für das in dem Reservoir PR befindliche Volumen erreicht, wird die Pumpe P2 angehalten.

Am Ende der Regenerationsphase, d.h. zu Beginn der zweiten bzw. folgenden Behandlungsphase beginnt die Pumpe P4 wieder mit der Förderung des Plasmas. Die Pumpe P2 fördert wieder mit der maximal möglichen Plasmaförderrate, was insbesondere dann der Fall ist, wenn die Pumpe P2 während der Regenerationsphase gestoppt wurde oder mit reduziertem Fluss betrieben wurde. Die maximal mögliche Förderrate der Pumpe P2 ergibt sich aus dem Blutfluss in dem extrakorporalen Kreislauf E1 und dem maximal abtrennbaren Plasmaanteil.

Die Förderrate der Pumpe P4 ist höher als bei der ersten Behandlungsphase. Die Pumpe P4 hat die Aufgabe, nicht nur das von der Pumpe P2 geförderte Plasma, sondern zusätzlich das in dem Reservoir PR angesammelte Plasma in der Behandlungsphase abzufördern.

Wird festgestellt, dass das Reservoir wieder entleert ist, beispielsweise durch Kontrolle der Waage BAL2, wird der Fluss der Pumpe P4 für die verbleibende Restzeit dieser Behandlungsphase auf den Fluss der Pumpe P2 reduziert, was zur Folge hat, dass kein Plasma in dem Reservoir angesammelt wird.

Ist der Adsorber A vollständig oder weitgehend beladen, d.h. ist die Aufnahmekapazität des Adsorbers A vollständig oder weitgehend erschöpft, wird die Behandlungsphase beendet und die Regenerationsphase eingeleitet.

Während der Regenerationsphase steht die Pumpe P4 und das Ventil V1 ist geschlossen, um sicherzustellen, dass keine Regenerationslösung in das Blut des Patienten gelangt. Mittels der Pumpe P3 und durch Ventilöffnungen der Ventile V3 und V4 werden in einer für den Adsorber A spezifischen Abfolge, Flussrate und Volumen Regenerations- und Spüllösung durch die Pumpe P3 über den Adsorber A in den Abfallbehälter 300 gefördert.

Wie dies aus Figur 3 weiter hervorgeht, sind das Reservoir R für Regenerationslösung und das Reservoir S für eine Spüllösung auf einer gemeinsamen Waage BAL1 angeordnet. Die Auswahl der Lösungen (Regenerationslösung oder Spüllösung) erfolgt mittels der Ventile V3 und V4. Die Förderrate der Pumpe P3 kann mit dem Signal der Waage BAL1 verglichen werden und auf diese Weise festgestellt werden, wie lange bzw. mit welcher Förderrate welche Lösung gefördert wird.

Um sicherzustellen, dass die richtige Lösung an den richtigen Zweig des Schlauchsystems angeschlossen wird, können konstruktiv nicht verwechselbare Konnektoren verwendet werden. Dies bedeutet, dass ein Teil des Konnektors nur mit einem ganz bestimmten Gegenstück verbunden werden kann.

Entsprechendes gilt für das Einlegen bzw. Aufrüsten des Schlauchsystems mit den Zuführungsschläuchen für die Regenerations- und Spüllösung. Auch dabei ist sicherzustellen, dass diese nicht vertauscht in die Ventile V3 und V4 eingelegt werden. Dies kann beispielsweise durch mechanisch unterschiedliche, nicht austauschbar konstruierte Druckabnahmestellen am Schlauchsystem und entsprechend gestaltete Aufnahmestellen geräteseitig gewährleistet werden. Unter einer "Druckabnahmestelle" ist der Bereich des Schlauches zu verstehen, der in das Ventil eingelegt wird.

Durch diese Maßnahme sowie durch die eindeutige Zuordnung der Konnektorelemente wird sichergestellt, dass die Zuordnung der Lösung bzw. des Reservoirs R und des Reservoirs S zu den Ventilen V3 und V4 korrekt ist.

Eine weitere Fehlerquelle ist die Leckage eines Ventils. So kann beispielsweise ein nicht vollständig geschlossenes Ventil V3 oder V4 dazu führen, dass ungewollt Regenerationslösung der Spüllösung bzw. dem Flüssigkeitsstrom beigemischt wird. Um sicherzustellen, dass die Ventile V3 und V4 korrekt arbeiten, kann vor dem Beginn der Förderung der Spüllösung, d.h. vor dem Öffnen des Ventils V4 mit der Pumpe P3 ein Unterdruck in dem Schlauchsegment zwischen der Pumpe P3 und den Ventilen V3 und V4 aufgebaut werden und die Waage BAL1 auf ein konstantes Gewicht hin überprüft werden. Wird eine Gewichtsänderung festgestellt, ist dies auf ein nicht vollständiges Schließen eines oder beide der Ventile V3 und V4 zurückzuführen.

Gleichzeitig wird auf der Druckseite der Pumpe P3 eine Überprüfung der Dichtigkeit, d.h. der Okklusion der Pumpe P4 sowie des Ventils V1. Die Überprüfung auf einen konstanten Druck erfolgt mittels des Drucksensors PS5.

Nach dem erfolgreichen Abschluss dieser Prüfungen werden die Ventile V2 und V4 geöffnet und die Spülung beginnt. Die Förderung der Spüllösung erfolgt mittels der Pumpe P3.

Alternativ oder zusätzlich können Ventile mit Überwachung des Schaltzustandes und/oder einer Überwachung auf einen korrekt eingelegten Schlauch bzw. Ventilsitz eingesetzt werden.

Wie dies aus Figur 3 hervorgeht, weist die Anordnung eine weitere Waage BAL3 auf, die das Gewicht des Abfallbehälters 300 erfasst. Aus einem Vergleich der durch die Waage BAL1 ermittelten Gewichtsabnahme und der durch die Waage BAL 3 ermittelten Gewichtszunahme kann geprüft werden, ob das Ventil V1 während der Spül- und Regenerationsphase geschlossen ist und die Pumpe P4 steht.

Eine entsprechende Prüfung kann auch die Ventile V2, V3 und V4 sowie für die Dichtigkeit, d.h. Okklusion der Pumpe P4 vorgenommen werden. Bei geschlossenen Ventilen V1 und V2 und stehender Pumpe P3 wird durch die Pumpe P4 ein Druck in dem Leitungssystem zwischen P4 und V1, V2 und P3 aufgebaut. Mittels des Drucksensors PS5, der sich in diesem Leitungssystem befindet, kann der Druck auf Konstanz geprüft werden. Ergibt sich ein konstanter Druck, wird das Ventil V1 geöffnet und die Behandlungsphase wird begonnen.

Eine zusätzliche Überprüfung des Ventils V2 während der Behandlungsphase erfolgt durch die Messung des Gewichts des Abfallreservoirs 300 mittels der Waage BAL3. Die Pumpe P3 wird in dieser Phase auf Stillstand überwacht.

Beim Übergang von Plasma zu Spüllösung wird für eine Übergangszeit der Flussweg vom Reservoir S für die Spüllösung zum extrakorporalen Kreislauf E2 freigeschaltet. Das Ventil V4 und V1 wird geöffnet und die Pumpe P3 wird aktiviert. Dieser Vorgang dauert so lange, bis das Plasma weitgehend aus dem Schlauchsystem und aus dem Adsorber A durch Spüllösung verdrängt worden ist.

Beim Wechsel von Spüllösung zu Plasma der Flussweg vom Reservoir PR über das Ventil V2 zum Abfall 300 freigeschaltet. Die Förderung des Plasmas wird in dieser Übergangsphase solange vorgenommen, bis die Spüllösung weitgehend aus dem Schlauchsystem und aus dem Adsorber A durch Plasma verdrängt worden ist.

In beiden Fällen kann die Sicherheit des Verfahrens durch eine Kombination der vorgenannten Überwachungen gewährleistet werden.

Durch die oben genannten Prüfungen wird sichergestellt, dass die Regenerationslösung nicht durch Spüllösung verdünnt und somit in ihrer Wirksamkeit beeinträchtigt wird, die Spüllösung nicht mit Regenerationslösung vermischt wird und dem Patienten somit in der nachfolgenden Behandlung nicht eine nicht-physiologisch zusammengesetzte Lösung zugeführt wird, dass die Regenerations- und/oder Spüllösung nicht mit Plasma im Reservoir vermischt wird und dem Patienten später wieder zugeführt wird, dass die Regenerations- und/oder Spüllösung nicht direkt dem Patienten zugeführt wird, kein unerkannter Plasmaverlust durch nicht ausreichend geschlossenes Ventil V2 erfolgt und die Regenerations- und/oder Spüllösung nicht durch Plasma verunreinigt wird.

Die Ausführungen zu der Betriebsweise der Anordnung gemäß Figur 3 gelten für sämtliche weiteren Ausführungsbeispiele der Figuren 1, 2 und 4 bis 8 entsprechend, soweit gleiche oder funktionsgleiche Elemente vorhanden sind.

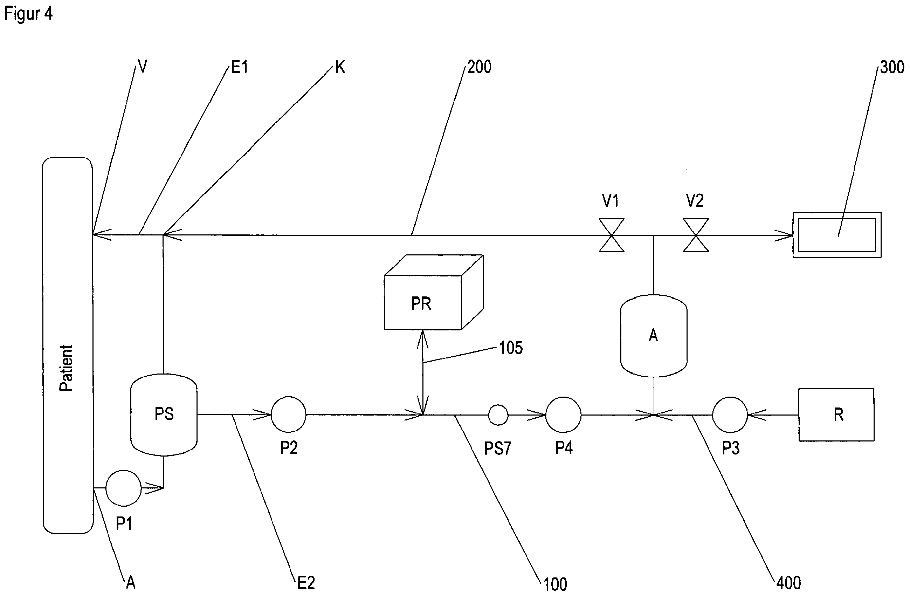

Das Flussschema gemäß Figur 4 entspricht dem zu Figur 1 mit dem Unterschied, dass sich das Reservoir PR nicht in der ersten Plasmaleitung 100 befindet, sondern in einer davon abzweigenden Leitung 105. Ein weiterer Unterschied zu der Anordnung gemäß Figur 1 besteht in der Anordnung eines Drucksensors PS7 zwischen dem Reservoir PR und der Plasmapumpe P4. Der Drucksensor PS7 hat die Aufgabe, eine grobe Fehlfunktion, wie z.B. die Okklusion des Beutelabflusses oder die Überfüllung des Beutels über das Nennvolumen hinaus festzustellen.

In den Anordnungen gemäß der Figuren 1 bis 4 erfolgt die Befüllung des Reservoirs PR jeweils mittels der zwischen dem Plasmaseparator PS und dem Reservoir in der ersten Plasmaleitung 100 angeordneten Pumpe P2 und die Entleerung des Reservoirs PR mittels der stromabwärts des Reservoirs PR in der Plasmaleitung 100 angeordneten Pumpe P4.

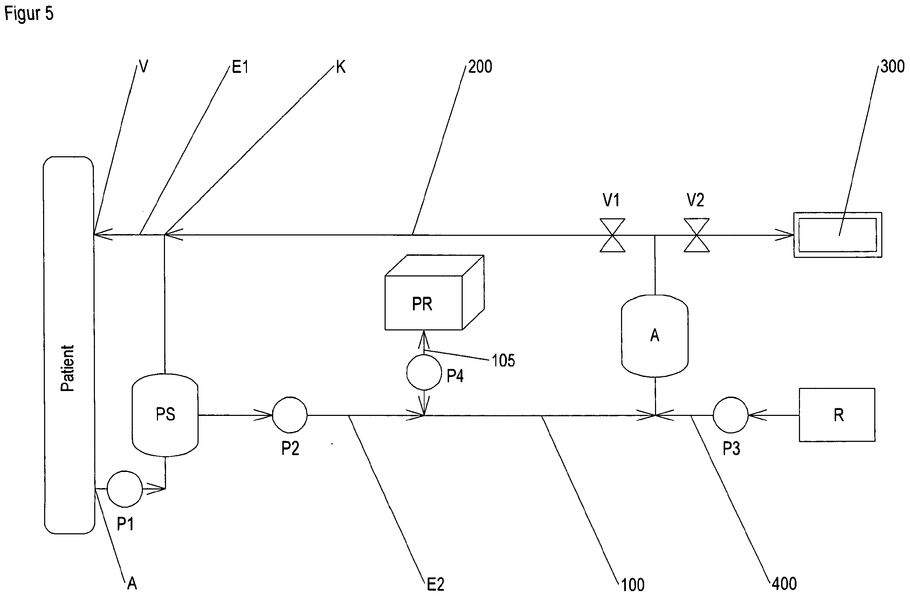

Abweichend davon ist das Reservoir PR und die Pumpe P4 zur Befüllung und zur Entleerung des Reservoirs PR in der Anordnung gemäß Figur 5 in einer von der ersten Plasmaleitung 100 abzweigenden Leitung 105 angeordnet. Die das Plasma von dem Plasmafilter PF fördernde Pumpe P2 befindet sich zwischen dem Plasmafilter PF und der Abzweigung der Leitung 105 von der ersten Plasmaleitung 100. Es ist eine einzige Leitung 105 vorgesehen, die zur Befüllung und Entleerung des Plasmareservoirs PR dient.

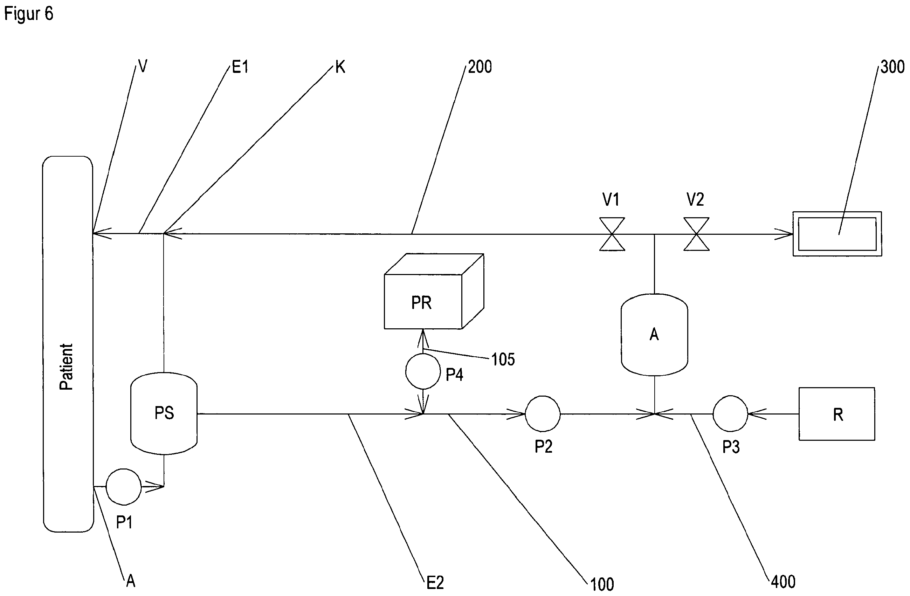

Die Anordnung gemäß Figur 6 entspricht der Anordnung gemäß Figur 5 mit dem Unterschied, dass die Pumpe P2 in der ersten Plasmaleitung zwischen der Abzweigung der Leitung 105 von der ersten Plasmaleitung 100 und dem Adsorber A angeordnet ist.

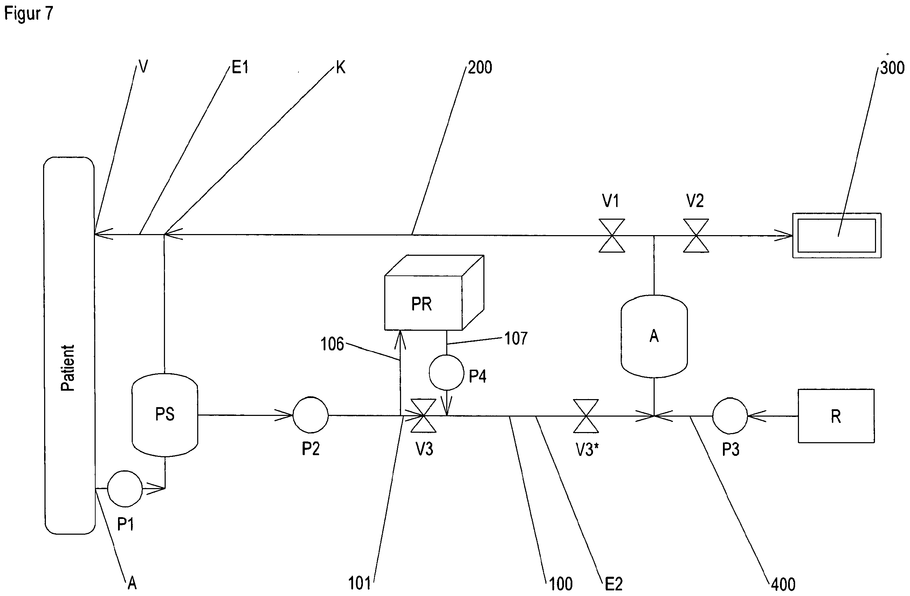

Aus dem Flussschema gemäß Figur 7 ist eine Anordnung ersichtlich, bei der das Reservoir PR in von der ersten Plasmaleitung 100 abzweigenden Leitungen angeordnet ist. Diese Leitungen sind durch die Bezugszeichen 106, 107 gekennzeichnet.

Die Leitung 106 dient zur Füllung des Reservoirs PR mittels der Pumpe P2, die zwischen dem Plasmafilter PF und der Abzweigung der Leitung 106 von der ersten Plasmaleitung 100 angeordnet ist. In der Leitung 107, die zur Entleerung des Reservoirs PR in die Plasmaleitung 100 mündet, befindet sich die Pumpe P4.

In dem Leitungsabschnitt 101 der ersten Plasmaleitung 100, der sich zwischen den beiden Abzweigungspunkten der Leitungen 106, 107 in die bzw. von der ersten Plasmaleitung 100 erstreckt, befindet sich das absperrbare Ventil V3. Bei der Befüllung des Reservoirs PR mit Plasma ist das Ventil V3 geschlossen, die Pumpe P2 läuft und die Pumpe P4 steht. Bei der Entleerung des Reservoirs PR ist das Ventil V3 geöffnet und die Pumpen P2 und P4 sind in Betrieb.

Das Absperrventil V3*, das ebenfalls in der ersten Plasmaleitung 100 angeordnet ist und sich zwischen den Einmündungspunkten der Leitungen 106, 107 und dem Adsorber A befindet, hat die Aufgabe, einen Zutritt von Regenerationslösung in die erste Plasmaleitung 100 und somit auch in das Reservoir PR zu verhindern.

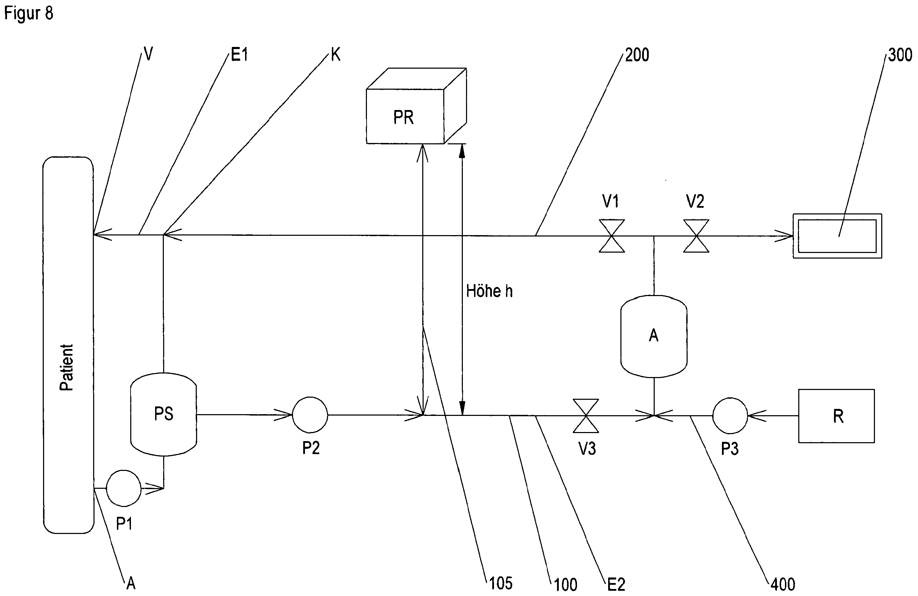

Die Anordnung gemäß Figur 8 zeichnet sich dadurch aus, dass sich das Reservoir PR in einer Abzweigungsleitung 105 befindet, wie dies auch in der Anordnung gemäß Figur 4 bis 7 der Fall ist. Abweichend von der Anordnung in diesen Figuren ist gemäß Figur 8 keine Pumpe zur Entleerung des Reservoirs PR vorgesehen. Die Befüllung des Reservoirs PR erfolgt bei geschlossenem Ventil V3 mittels der Plasmapumpe P2, die zwischen dem Plasmafilter PF und der Abzweigung der Leitung 105 von der ersten Plasmaleitung angeordnet ist. Die Entleerung des Reservoirs erfolgt mittels der Schwerkraft, wozu eine gewisse Höhe h erforderlich ist.

In den Figuren ist ein Reservoir R für eine Regenerationslösung vorhanden, das über eine Leitung 400, in der sich eine Pumpe P3 befindet, mit der ersten Plasmaleitung 100 verbunden ist. Zum Zwecke der Regeneration des Adsorbers wird die Leitung 100 und die Rückführleitung 200 gegen das Eintreten von Regenerationslösung geschlossen und das Ventil V2 wird geöffnet, so dass die verbrauchte Regenerationslösung in den Abfall 300 gelangen kann. Ist die Regenerationsphase abgeschlossen, wird die Pumpe P3 gestoppt und das Ventil V2 geschlossen.

## Patentansprüche

1. Vorrichtung zur Durchführung einer Apheresebehandlung, wobei
die Vorrichtung einen extrakorporalen Kreislauf (E2) aufweist, in dem sich ein regenerierbarer Einfach-Adsorber (A) zur Abtrennung von Substanzen aus Blut oder zur Abtrennung von Substanzen aus mittels eines Plasmaseparators (PS) gewonnenen Plasma befindet,
eine Leitung (100) zur Führung des Blutes oder des Plasmas vorgesehen ist, die sich zu dem Adsorber (A) erstreckt und über die der Adsorber (A) mit Blut oder Plasma beaufschlagt wird,
ein Reservoir (PR) zur Aufnahme von Blut oder Plasma vorgesehen ist, das stromaufwärts des Adsorbers (A) in der Leitung (100) angeordnet ist oder stromaufwärts des Adsorbers (A) mit der Leitung (100) in Verbindung steht, und
ein Controller oder Schaltmittel vorgesehen ist, der/das derart ausgebildet ist, dass das Reservoir (PR) mit Blut oder Plasma befüllt wird und die Beaufschlagung des Adsorbers (A) mit Blut oder Plasma unterbunden wird, wenn die Regeneration des Adsorbers (A) durchgeführt wird,
**dadurch gekennzeichnet, dass**
eine Bypassleitung um den Adsorber (A) vorgesehen ist, und
der Controller oder die Schaltmittel derart ausgebildet sind, dass dieser/diese die Förderung des Blutes oder des Plasmas durch die Bypassleitung veranlasst, wenn die maximale Füllmenge in dem Reservoir (PR) erreicht ist.

2. Vorrichtung nach Anspruch 1, **dadurch gekennzeichnet, dass** ein erster extrakorporaler Kreislauf (E1) vorgesehen ist, in dem sich der Plasmaseparator (PS) befindet, an dessen Sekundärseite sich ein zweiter extrakorporaler Kreislauf (E2) anschließt, wobei das Reservoir (PR) und der Adsorber (A) in dem zweiten extrakorporalen Kreislauf (E2) angeordnet sind und wobei eine Rücklaufleitung (200) vorgesehen ist, die den Adsorber (A) auslassseitig mit dem ersten extrakorporalen Kreislauf (E1) verbindet.

3. Vorrichtung nach Anspruch 1 oder 2, **dadurch gekennzeichnet, dass** Mittel zur Entleerung des Reservoirs (PR) vorgesehen sind und dass der Controller oder Schaltmittel mit den Mitteln zur Entleerung des Reservoirs (PR) derart in Verbindung stehen, dass diese durch den Controller oder die Schaltmittel aktivierbar sind, wenn die Regenerationsphase des Adsorbers (A) abgeschlossen ist.

4. Vorrichtung nach Anspruch 3, wobei die Mittel Pumpen oder Ventile sind.

5. Vorrichtung nach einem der vorhergehenden Ansprüche, **dadurch gekennzeichnet, dass** die Mittel zur Befüllung des Reservoirs (PR) durch eine stromabwärts des Plasmaseparators (PS) angeordnete Pumpe, durch die Blutpumpe, durch eine Pumpe, die in einer zu dem Reservoir (PR) führenden Leitung (100) angeordnet ist, oder durch einen pneumatischen Aktor gebildet werden.

6. Vorrichtung nach einem der vorhergehenden Ansprüche, **dadurch gekennzeichnet, dass** Mittel zur Entleerung des Reservoirs (PR) durch die Schwerkraft, durch eine stromabwärts des Reservoirs (PR) angeordnete Pumpe, durch eine Pumpe, die in einer von dem Reservoir (PR) abführenden Leitung (100) angeordnet ist, durch einen pneumatischen Aktor oder durch das elastische Material des Reservoirs (PR) gebildet werden.

7. Vorrichtung nach einem der vorhergehenden Ansprüche, **dadurch gekennzeichnet, dass** in der Leitung (100) eine Pumpe angeordnet ist und dass sich das Reservoir (PR) zwischen der Pumpe und dem Adsorber (A) befindet.

8. Vorrichtung nach einem der vorhergehenden Ansprüche, wobei das Reservoir (PR) in der Leitung, in einer von der Leitung abzweigenden Leitung oder in einer zur Leitung parallel verlaufenden Leitung angeordnet ist

9. Vorrichtung nach einem der vorhergehenden Ansprüche, **dadurch gekennzeichnet, dass** es sich bei dem Reservoir (PR) um einen starren Hohlkörper oder um einen Hohlkörper mit elastischen und/oder flexiblen Wandungen handelt.

10. Vorrichtung nach einem der vorhergehenden Ansprüche, wobei vorgesehen ist, dass das Reservoir (PR) über eine Be- und Entlüftung verfügt.

11. Vorrichtung nach einem der vorhergehenden Ansprüche, wobei das Reservoir (PR) über einen Abfluss und über einen Zufluss verfügt, die durch getrennte Elemente oder durch ein und dasselbe Element gebildet werden.

12. Vorrichtung nach einem der vorhergehenden Ansprüche, wobei das Reservoir (PR) ein Volumen zwischen 10 ml und 1000 ml aufweist

13. Vorrichtung nach einem der vorhergehenden Ansprüche, **dadurch gekennzeichnet, dass** Mittel zur Ermittlung und/oder Überwachung und/oder Begrenzung der Menge des in dem Reservoir (PR) befindlichen Blutes oder Plasmas vorgesehen sind.

14. Vorrichtung nach Anspruch 13, **dadurch gekennzeichnet, dass** die Mittel durch einen Drucksensor, durch einen optischen Sensor, durch einen Ultraschallsensor, durch einen Wegaufnehmer, durch ein festes Gehäuse zur Volumenbegrenzung, durch eine Waage oder durch eine Flussmesseinrichtung zur Messung des Zuflusses und des Abflusses aus dem Reservoir (PR) gebildet werden.

15. Vorrichtung nach einem der vorhergehenden Ansprüche, **dadurch gekennzeichnet, dass** der Controller oder die Schaltmittel derart ausgebildet sind, dass die Aussetzung des Betriebes der Blutpumpe oder des Plasmaseparators (PS) bewirkt werden kann, wenn die maximale Füllmenge in dem Reservoir (PR) erreicht ist.

## Claims

1. An apparatus for carrying out an apheresis treatment, wherein
the apparatus has an extracorporeal circuit (E2) in which a regenerable single adsorber (A) is located for separating substances from blood or for separating substances from plasma acquired by means of a plasma separator (PS),
a line (100) is provided for conducting the blood or the plasma which extends to the adsorber (A) and via which blood or plasma is applied to the adsorber (A),
a reservoir (PR) is provided for receiving blood or plasma and is arranged upstream of the adsorber (A) in the line (100) or is in communication with the line (100) upstream of the adsorber (A); and
a controller or switching means is provided which is configured such that the reservoir (PR) is filled with blood or plasma and the application of blood or plasma to the adsorber (A) is suppressed when the regeneration of the adsorber (A) is carried out,
**characterized in that**
a bypass line about the adsorber (A) is provided; and
the controller or the switching means is configured such that it instigates the conveying of the blood or of the plasma through the bypass line when the maximum filling quantity in the reservoir (PR) has been reached.

2. An apparatus in accordance with claim 1, **characterized in that** a first extracorporeal circuit (E1) is provided in which the plasma separator (PS) is located whose secondary side is adjoined by a second extracorporeal circuit (E2), wherein the reservoir (PR) and the adsorber (A) are arranged in the second extracorporeal circuit (E2), and wherein a return line (200) is provided which connects the adsorber (A) to the first extracorporeal circuit (E1) at the outlet side.

3. An apparatus in accordance with claim 1 or claim 2, **characterized in that** means are provided for emptying the reservoir (PR); and **in that** the controller or switching means are in communication with the means for emptying the reservoir (PR) such that they can be actuated by the controller or by the switching means when the regeneration phase of the adsorber (A) has ended.

4. An apparatus in accordance with claim 3, wherein the means are pumps or valves.

5. An apparatus in accordance with one of the preceding claims, **characterized in that** the means for filling the reservoir (PR) are formed by a pump arranged downstream of the plasma separator (PS), by the blood pump, by a pump which is arranged in a line (100) leading to the reservoir (PR) or by a pneumatic actuator.

6. An apparatus in accordance with one of the preceding claims, **characterized in that** the means for emptying the reservoir (PR) are formed by gravity, by a pump arranged downstream of the reservoir (PR), by a pump which is arranged in a line (100) leading off from the reservoir (PR), by a pneumatic actuator or by the elastic material of the reservoir (PR).

7. An apparatus in accordance with one of the preceding claims, **characterized in that** a pump is arranged in the line (100) and that the reservoir (PR) is located between the pump and the adsorber (A).

8. An apparatus in accordance with one of the preceding claims, wherein the reservoir (PR) is arranged in the line, in a line branching off from the line or in a line extending in parallel with the line.

9. An apparatus in accordance with one of the preceding claims, **characterized in that** the reservoir (PR) is a rigid hollow body or a hollow body having elastic and/or flexible walls.

10. An apparatus in accordance with one of the preceding claims, wherein provision is made that the reservoir (PR) has a ventilation or bleeding device.

11. An apparatus in accordance with one of the preceding claims, wherein the reservoir (PR) has an outflow and an inflow which are formed by separate elements or by one and the same element.

12. An apparatus in accordance with one of the preceding claims, wherein the reservoir (PR) has a volume between 10 ml and 1000 ml.

13. An apparatus in accordance with one of the preceding claims, **characterized in that** means are provided for determining and/or monitoring and/or limiting the quantity of the blood or plasma located in the reservoir (PR).

14. An apparatus in accordance with claim 13, **characterized in that** the means are formed by a pressure sensor, by an optical sensor, by an ultrasound sensor, by a position encoder, by a fixed housing for volume limitation, by a set of scales or by a flow measuring device for measuring the inflow into and the outflow out of the reservoir (PR).

15. An apparatus in accordance with one of the preceding claims, **characterized in that** the controller or the switching means are configured such that the suspension of the operation of the blood pump or of the plasma separator (PS) can be effected when the maximum filling quantity in the reservoir (PR) has been reached.

## Revendications

1. Dispositif de réalisation d'un traitement par aphérèse, dans lequel
le dispositif comporte un circuit extracorporel (E2), dans lequel se trouve un adsorbant (A) simple régénérable servant à séparer des substances du sang ou à séparer des substances de plasma obtenu au moyen d'un séparateur de plasma (PS),
une conduite (100) est prévue pour guider le sang ou le plasma, laquelle s'étend jusqu'à l'adsorbant (A) et par le biais de laquelle l'adsorbant (A) est alimenté en sang ou plasma,
un réservoir (PR) est prévu pour la réception du sang ou du plasma, ledit réservoir étant disposé en amont de l'adsorbant (A) dans la conduite (100) ou étant en liaison avec la conduite (100) en amont de l'adsorbant (A), et
un contrôleur ou un moyen de commutation est prévu, qui est conçu de telle manière que le réservoir (PR) est rempli de sang ou de plasma et que l'alimentation de l'adsorbant (A) en sang ou plasma est empêchée quand la régénération de l'adsorbant (A) est effectuée,
**caractérisé en ce que**
une conduite de dérivation est prévue pour contourner l'adsorbant (A), et
le contrôleur ou les moyens de commutation sont conçus de telle manière que celui-ci/ceux-ci provoque(nt) le transport du sang ou du plasma par la conduite de dérivation, quand la quantité de remplissage maximale est atteinte dans le réservoir (PR).

2. Dispositif selon la revendication 1, **caractérisé en ce qu'**un premier circuit extracorporel (E1) est prévu, dans lequel se trouve le séparateur de plasma (PS), au côté secondaire duquel est raccordé un second circuit extracorporel (E2), le réservoir (PR) et l'adsorbant (A) étant disposés dans le second circuit extracorporel (E2) et une conduite de retour (200) étant prévue, qui relie l'adsorbant (A) côté sortie au premier circuit extracorporel (E1).

3. Dispositif selon la revendication 1 ou 2, **caractérisé en ce que** des moyens de vidage du réservoir (PR) sont prévus et **en ce que** le contrôleur ou les moyens de commutation sont en liaison avec les moyens de vidage du réservoir (PR) de telle manière que ces derniers peuvent être activés par le contrôleur ou les moyens de commutation quand la phase de régénération de l'adsorbant (A) est terminée.

4. Dispositif selon la revendication 3, dans lequel les moyens sont des pompes ou des soupapes.

5. Dispositif selon l'une des revendications précédentes, **caractérisé en ce que** les moyens de remplissage du réservoir (PR) sont formés par une pompe disposée en aval du séparateur de plasma (PS), par la pompe à sang, par une pompe, qui est disposée dans une conduite (100) allant vers le réservoir (PR), ou par un actionneur pneumatique.

6. Dispositif selon l'une des revendications précédentes, **caractérisé en ce que** des moyens de vidage du réservoir (PR) sont formés par la gravité, par une pompe disposée en aval du réservoir (PR), par une pompe, qui est disposée dans une conduite (100) partant du réservoir (PR), par un actionneur pneumatique ou par le matériau élastique du réservoir (PR).

7. Dispositif selon l'une des revendications précédentes, **caractérisé en ce qu'**une pompe est disposée dans la conduite (100) et **en ce que** le réservoir (PR) se trouve entre la pompe et l'adsorbant (A).

8. Dispositif selon l'une des revendications précédentes, dans lequel le réservoir (PR) est disposé dans la conduite, dans une conduite bifurquant de la conduite ou dans une conduite parallèle à la conduite.

9. Dispositif selon l'une des revendications précédentes, **caractérisé en ce que** le réservoir (PR) est un corps creux rigide ou un corps creux doté de parois élastiques et/ou flexibles.

10. Dispositif selon l'une des revendications précédentes, dans lequel le réservoir (PR) est prévu disposant d'une aération et d'une évacuation d'air.

11. Dispositif selon l'une des revendications précédentes, dans lequel le réservoir (PR) dispose d'un système d'écoulement et d'un système d'afflux, qui sont formés par des éléments séparés ou par un seul et même élément.

12. Dispositif selon l'une des revendications précédentes, dans lequel le réservoir (PR) présente un volume compris entre 10 ml et 1000 ml.

13. Dispositif selon l'une des revendications précédentes, **caractérisé en ce que** des moyens de détection et/ou de surveillance et/ou de limitation de la quantité du sang ou plasma se trouvant dans le réservoir (PR) sont prévus.

14. Dispositif selon la revendication 13, **caractérisé en ce que** les moyens sont formés par un capteur de pression, par un capteur optique, par un capteur à ultrasons, par un capteur de déplacement, par un boîtier solide pour la limitation du volume, par une balance ou par un dispositif de mesure du débit destiné à la mesure de l'afflux ou de l'écoulement du réservoir (PR).

15. Dispositif selon l'une des revendications précédentes, **caractérisé en ce que** le contrôleur ou les moyens de commutation sont réalisés de telle manière que l'interruption du fonctionnement de la pompe à sang ou du séparateur de plasma (PS) peut être provoquée quand la quantité de remplissage maximale est atteinte dans le réservoir (PR).
